# EUROPÄISCHE PATENTSCHRIFT

(11) **EP 2 334 271 B1**
(45) Veröffentlichungstag und Bekanntmachung des Hinweises auf die Patenterteilung: **08.04.2015**
(21) Anmeldenummer: 08801693.6
(22) Anmeldetag: 25.08.2008
(51) Int. Cl.: A61F 9/008, A61F 9/009

(54) **ANKOPPLUNG EINES AUGES AN EINE LASEREINRICHTUNG**
COUPLING OF AN EYE TO A LASER DEVICE
Couplage d'un oeil à un dispositif laser

(43) Veröffentlichungstag der Anmeldung: 22.06.2011
(73) Patentinhaber: WaveLight GmbH, 91058 Erlangen (DE)
(72) Erfinder: KITTELMANN, Olaf, 14163 Berlin (DE); LI, Jing, 91052 Erlangen (DE); ROBL, Gerhard, 90547 Stein (DE); VOGLER, Klaus, 90542 Eckental (DE); ZERL, Bernd, 91080 Uttenreuth (DE); DEISINGER, Thomas, 90556 Cadolzburg (DE)
(74) Vertreter: Katérle, Axel
(86) Internationale Anmeldenummer: PCT/EP2008/006962
(87) Internationale Veröffentlichungsnummer: WO 2010/022745

(56) Entgegenhaltungen:
- EP-A- 1 570 822
- EP-A1- 1 970 034
- DE-A1- 10 354 025
- US-A1- 2004 243 112
- US-A1- 2007 093 795
- US-A1- 2008 071 254

## Beschreibung

Die Erfindung befasst sich allgemein mit der Herstellung einer mechanischen Kopplung zwischen einem biologischen Gewebe und einer Lasereinrichtung, deren Laserstrahlung zur Behandlung des Gewebes genutzt wird. Im speziellen befasst sich die Erfindung mit der Ankopplung einer Lasereinrichtung an ein Auge, insbesondere ein menschliches Auges, um mit der Laserstrahlung einen oder mehrere Schnitte in das Auge einzubringen.

Für eine gezielte Wirkung der Laserstrahlung ist eine präzise Lokalisierung des Strahlfokus relativ zu dem zu behandelnden Gewebe unerlässlich. Insbesondere für die Erzeugung von Schnitten im Gewebe wird häufig ein verhältnismäßig kleiner Strahlfokus angestrebt, um die Schnittdicke gering zu halten. Entsprechend präzise muss dann auch die Positionierung des Strahlfokus sein. Dies gilt insbesondere für die Einbringung von Schnitten in Augengewebe, wie sie beispielsweise im Rahmen der sogenannten Fs-LASIK vorkommen. LASIK steht für Laser In-situ Keratomileusis und bezeichnet eine Technik zur Behandlung von Fehlsichtigkeiten, bei der zunächst aus dem vorderen Bereich der Kornea ein Deckelscheibchen (in der Fachwelt regelmäßig als Flap bezeichnet) herausgeschnitten wird, das zum Teil noch mit der Kornea verbunden bleibt, so dass es für eine anschließende Ablation von darunter liegendem Korneagewebe mittels Laserstrahlung zur Seite geklappt werden kann. Nach Durchführung der Ablation (Gewebeabtrag) wird der Flap zurückgeklappt und es erfolgt eine relative schnelle Heilung unter weitgehender Unversehrtheit der Korneaoberfläche.

Für die Erzeugung des Flaps verwendet eine bisher gängige Technik einen mechanischen Schneidhobel (Mikrokeratom), welcher mit einer schnell oszillierenden Schneide von der Seite her in die Kornea einschneidet. Es wird seit einiger Zeit auch an Systemen gearbeitet, die eine Flaperzeugung mittels fokussierter Laserstrahlung mit Pulsdauern im Femtosekundenbereich gestatten. Daher der Name Femtosekunden- oder Fs-LASIK. Die Strahlung wird dabei unter der Vorderfläche der Kornea im Inneren des Gewebes fokussiert und die Fokuspunkte werden in der gewünschten Fläche so positioniert, dass im Ergebnis ein Flap aus der Kornea herausgeschnitten wird.

Gewebeschnitte im Auge werden aber nicht nur bei der Fs-LASIK benötigt, sondern auch bei anderen Indikationen, etwa bei der Keratoplastik (z.B. anteriore oder posteriore lamellare Keratoplastik, penetrierende Keratoplastik bei Hornhauttransplantationen), der Fs-Lentikel-Extraktion zur Refraktionskorrektur, beim Schneiden von interkornealen Ringsegmenten zur Stabilisierung von Keratokonus und Hornhautvorwölbung (z.B. für das Einsetzen von Intacs, d.h. kleinen implantierten Ringsegmenten zur biomechanischen Stabilisierung der Hornhaut), bei Kataraktinzisionen, bei Presbyopie-Schnitten in der Augenlinse, bei intrastromalen Inlays, bei der Keratomie für Astigmatismen, bei der kornealen Resektion und dgl.

Es ist im Stand der Technik bekannt (siehe z.B. US 5,549,632, WO 03/002008 A1), bei Augenlasereinrichtungen eine planparallele Applanationslinse auf die Kornea aufzudrücken. Durch das Aufdrücken der Applanationslinse verformt sich das Auge und schmiegt sich flächig an die augenzugewandte Unterseite der Applanationslinse an. Der Strahlfokus der Laserstrahlung ist in z-Richtung gegenüber der Applanationslinse referenziert (mit z-Richtung ist hierbei die Strahllängsrichtung gemeint). Durch das Anliegen des Auges an der Applanationslinse ist ein fester z-Bezug zwischen Auge und Linse gegeben, was eine präzise z-Positionierung des Strahlfokus in beliebigen Regionen in der Kornea oder anderen Gewebestrukturen in der Tiefe des Auges gestattet.

Neben planparallelen Applanationslinsen sind im Stand der Technik auch Linsen (oder allgemein Kontaktgläser) mit sphärisch, asphärisch oder anderweitig gekrümmten Flächen bekannt geworden, die gleichermaßen eine z-Referenzierung ermöglichen. Durch geeignet konkave Gestaltung der augenzugewandten Linsenunterseite kann die Verformung des Auges beim Aufsetzen der Linse verringert werden. Dies ist insofern vorteilhaft, als sich der Augeninnendruck nicht so sehr erhöht wie bei einer aufgedrückten Applanationslinse mit planer Unterseite. Allerdings verschlechtern die gekrümmten Linsenflächen die Fokussierbarkeit der Laserstrahlung.

Um das Auge des Patienten in fester Distanz zur Fokussieroptik der Lasereinrichtung zu halten, werden im Stand der Technik in der Regel Saugringe verwendet, welche mittels Unterdruck an die Sclera des Auges angesaugt werden und die Kornea ringförmig umgeben. Entweder ist die Applanationslinse dabei in den Saugring integriert, wie beispielsweise in Fig. 4C der oben erwähnten US 5,549,632 gezeigt. Oder es ist die Applanationslinse Teil einer gesonderten Komponente, welche mit dem Saugring gekoppelt wird, siehe beispielsweise Fig. 7 von WO 03/002008 A1, wo die Applanationslinse fest an einem Kegelkörper angebracht ist, der im Bereich seiner Kegelbasis zur Ankopplung an die Fokussieroptik einer Lasereinrichtung ausgeführt ist und an seinem schmalen Kegelende mittels einer gesonderten Klemmzange in feste Kopplung mit dem Saugring gebracht werden kann. Aus US 2007/0093795 A1 ist ein weiteres Ausführungsbeispiel einer Vorrichtung zur Positionierung eines Patientenauges gegenüber einem Lasergerät bekannt wobei die Vorrichtung einen Saugring_ sowie eine von diesem gesondert ausgebildete in dem Dokument als Patienteninterface bezeichnete Komponente umfasst die ein Kontaktelement mit einer konkaven Anlagefläche für das Auge aufweist und durch Saugkraft an einer Ausrichtkomponente des Lasergeräts fixierbar ist.

Die dreiteilige Konstruktion gemäß WO 03/002008 A1 mit einem Saugring, einer Klemmzange und einem die Applanationslinse tragenden Kegelkörper gestattet eine voneinander unabhängige Heranführung der Bearbeitungsoptik der Lasereinrichtung und des Patientenauges bis nahe aneinander. Der Saugring sitzt dabei schon auf dem Auge, während der Kegel schon an der Bearbeitungsoptik angebracht ist. Sind die Bearbeitungsoptik und das Patientenauge nahe genug aneinander herangebracht, kommt die Klemmzange zum Einsatz, welche die mechanische Kopplung zwischen dem Kegel und dem Saugring herstellt.

Es ist leicht nachvollziehbar, dass die mechanische Fixierung des Auges gegenüber der Bearbeitungsoptik mittels der Klemmzange eine kritische Phase der Operationsvorbereitung ist. Die dabei wirkenden Druck- und Scherkräfte dürfen am Patientenauge nicht zu Verletzungen führen oder durch ungenaue Positionierung der beteiligten mechanischen Komponenten zueinander eine übermäßige und möglicherweise gefährliche Erhöhung des Augeninnendrucks bewirken. Tritt über einen längeren Zeitraum ein erhöhter Augeninnendruck auf, kann dies unter Umständen zu einer Schädigung des Sehnervs führen. Selbst wenn es möglich ist, die Optik mittels eines Steuerknüppels (Joystick) mehr oder weniger präzise zu positionieren, ist der Kegel dennoch starr mit der Optik verbunden, weswegen der letztendlich herbeigeführte mechanische Kontakt zum Auge und die damit verbundene Einebnung des Auges starr bleiben. Die hierbei auf das Auge ausgeübten Kräfte, und zwar sowohl die beim Vorgang der Ankopplung auftretenden Kräfte als auch die nach erfolgter Applanation wirkenden Kräfte, sind kaum vorhersehbar und können von Patient zu Patient unterschiedlich sein.

Insgesamt ist für zahlreiche im Stand der Technik bekannte Lösungen charakteristisch, dass eine Applanationslinse, oder allgemeiner ausgedrückt, ein Kontaktglas, gleich welcher Form, im Zuge der Ankopplung des Auges an die Bearbeitungsoptik auf das Auge aufgedrückt wird, und zwar so stark, dass sich das Auge in dem für die nachfolgende Bearbeitung erforderlichen Bereich an das Kontaktglas anschmiegt. Dieses Aufdrücken des Kontaktglases auf das Auge ist regelmäßig mit Druckstößen verbunden, die vom Patienten als unangenehm empfunden werden können. Das Auge wird dabei gequetscht und kann unter Umständen Schaden nehmen, insbesondere weil die auftretenden Zug-, Druck- und Scherkräfte nicht genau vorherbestimmbar sind.

Aufgabe der Erfindung ist es, eine schonendere Ankopplung eines Auges, an eine Lasereinrichtung zu ermöglichen.

Zur Lösung dieser Aufgabe ist das in Anspruch 1 definierte Verfahren vorgesehen.

Bei der erfindungsgemäßen Lösung werden die Schnittstelleneinheit und die Stabilisierungskomponente zunächst einander angenähert, bis sie eine vorgegebene erste Relativstellung erreichen. Diese relative Annäherung kann beispielsweise mittels motorischer Antriebsmittel oder händisch erfolgen. Sobald die erste Relativstellung erreicht ist, beginnt die Evakuierung einer Saugkammer, welche zwischen der Schnittstelleneinheit und der Stabilisierungskomponente gebildet ist, aber auch zum Teil von der Oberfläche des Gewebes begrenzt ist. Die Evakuierung der Saugkammer sorgt infolgedessen für ein gegenseitiges Ansaugen der Schnittstelleneinheit und der Stabilisierungskomponente, was diese aneinander hält. Der Unterdruck wirkt aber auch auf Teile der Gewebeoberfläche. Dies wird erfindungsgemäß genutzt, um das Gewebe gegen eine von der Schnittstelleneinheit gebildete Gewebeanlagefläche zu saugen, so dass dort eine flächige Anschmiegung des Gewebes an die Gewebeanlagefläche stattfindet. In der ersten Relativstellung der Schnittstelleneinheit und der Stabilisierungskomponente, bevor die Evakuierung der Saugkammer begonnen wird, besteht bei einer Ausgestaltung der Erfindung noch kein Kontakt zwischen dem Gewebe und der Gewebeanlagefläche. Gemäß einer alternativen Ausgestaltung kann schon ein solcher Kontakt bestehen, der jedoch durch die Evakuierung der Saugkammer signifikant vergrößert wird. Die Geometrie der Schnittstelleneinheit und der Stabilisierungskomponente sowie die Stärke des in der Saugkammer erzeugten Unterdrucks sind derart, dass nach erfolgter Evakuierung das Gewebe in einem Bereich an der Gewebeanlagefläche anliegt, der mindestens so groß wie die Ausdehnung des beabsichtigten Behandlungsbereichs ist, jedoch zu Beginn der Evakuierung noch signifikant kleiner als dieser Behandlungsbereich ist.

Insbesondere bewirkt die Evakuierung der Saugkammer eine Vergrößerung des Kontaktbereichs zwischen dem Gewebe und der Gewebeanlagefläche auf mindestens das Eineinhalbfache, vorzugsweise auf mindestens das Doppelte, und höchstvorzugsweise um ein Mehrfaches. Im Fall eines näherungsweise kreisscheibenförmigen Kontaktbereichs zwischen dem Gewebe und der Gewebeanlagefläche, wie er beispielsweise im Fall eines Auges regelmäßig vorkommt, sollte die Evakuierung der Saugkammer eine Vergrößerung des Durchmessers des Kontaktbereichs um mindestens 30 Prozent, vorzugsweise um mindestens 50 Prozent, noch bevorzugter um mindestens 70 Prozent und höchstbevorzugt um mindestens 90 Prozent bewirken.

Im Unterschied zu bekannten Lösungen auf dem Gebiet der laserchirurgischen Ophthalmologie setzt die Erfindung nicht allein auf die Einebnung oder anderweitige Formung des zu behandelnden Gewebes durch Aufdrücken eines Kontaktelements auf das Gewebe, sondern auf die Erzeugung einer Saugwirkung auf die betreffenden Teile der Gewebeoberfläche, durch die das Gewebe gegen die Gewebeanlagefläche gesogen wird. Diese grundlegend andere Wirkungsweise gestattet es, Druckstöße und Quetschungen, wie sie beim vollständigen Aufdrücken eines Kontaktelements unvermeidbar sind, zu vermeiden oder - sofern zu Beginn der Evakuierung schon ein gewisser kleinflächiger Kontakt zwischen Gewebe und Gewebeanlagefläche vorhanden ist - zumindest stark zu reduzieren. Es treten keine oder zumindest nur in äußerst geringem Maße unkontrollierte Kräfte auf (auch nicht vorübergehend). Weil die Saugkraft, die über die Stärke des Unterdrucks definiert und reproduzierbar einstellbar ist, vertikal von der Gewebeoberfläche weg wirkt, treten keine seitlichen Scherkräfte auf, die beim Auge leicht zu Epithelschädigungen führen könnten.

Ein weiterer Vorteil der erfindungsgemäßen Lösung besteht in der problemlosen Abkopplung der Schnittstelleneinheit von der Stabilisierungskomponente im Fall von Komplikationen oder einer Panikreaktion des Patienten. Durch Belüftung der Saugkammer kann die Kopplung zwischen Schnittstelleneinheit und Stabilisierungskomponente und damit zwischen Gewebe und Lasereinrichtung instantan, d.h. ohne Verzögerung, freigegeben werden.

Im Fall von Augenbehandlungen wird durch das Ansaugen der Kornea an die Anlagefläche der Schnittstelleneinheit der Innendruck des Auges, wenn überhaupt, nur geringfügig erhöht. Dies lässt die Operation den Patienten als angenehmer empfinden.

Gemäß einer Weiterbildung der Erfindung kann die relative Annäherung der Schnittstelleneinheit an die Stabilisierungskomponente in einer Richtung erfolgen, längs der über die erste Relativstellung hinaus eine weitere relative Annäherung der Schnittstelleneinheit und der Stabilisierungskomponente insbesondere bis in einen Zustand wechselseitigen Anschlags möglich ist. Das heißt, die erste relative Annäherung der Schnittstelleneinheit und der Stabilisierungskomponente wird gestoppt, noch bevor beide maximal einander angenähert sind. In der ersten Relativstellung kann dementsprechend bei einer möglichen Ausführungsform noch ein gewisser Abstand zwischen der Schnittstelleneinheit und der Stabilisierungskomponente in Richtung der Annäherungsbewegung vorhanden sein. Durch die anschließende Evakuierung der Saugkammer kann sich dieser Abstand verringern, nämlich in der Weise, dass die Evakuierung der Saugkammer eine relative Bewegung der Schnittstelleneinheit und die Stabilisierungskomponente in eine zweite Relativstellung bewirkt, in der sie stärker einander angenähert sind als in der ersten Relativstellung. Zu beachten ist, dass diese weitere relative Annäherung der Schnittstelleneinheit und der Stabilisierungskomponente über die erste Relativstellung hinaus allein durch den in der Saugkammer herrschenden Unterdruck bewirkt wird und nicht durch eine externe motorische oder händische Krafteinwirkung auf die Schnittstelleneinheit oder/und die Stabilisierungskomponente. Insbesondere bewirkt der Unterdruck in der Saugkammer eine gegenseitige Anziehung der Schnittstelleneinheit und der Stabilisierungskomponente in eine Anschlagstellung, in der keine weitere Annäherung mehr möglich ist.

Der relative Bewegungsweg der Schnittstelleneinheit und der Stabilisierungskomponente zwischen der ersten und der zweiten Relativstellung ist zweckmäßigerweise vergleichsweise gering und beträgt vorzugsweise nicht mehr als 1 mm. Insbesondere wird durch das Evakuieren der Saugkammer eine weitere Annäherung der Schnittstelleneinheit und der Stabilisierungskomponente um deutlich weniger als einen Millimeter bewirkt, beispielsweise nur um zwei oder drei Zehntel Millimeter. In jedem Fall empfiehlt es sich, dass ein auf die relative Annäherung der Schnittstelleneinheit und der Stabilisierungskomponente von der ersten in die zweite Relativstellung zurückzuführender Anteil an der Vergrößerung des Kontaktbereichs zwischen dem Gewebe und der Gewebeanlagefläche klein, insbesondere vernachlässigbar klein im Vergleich zu einem auf das Ansaugen des Gewebes an die Gewebeanlagefläche zurückzuführenden Anteil ist. Anders ausgedrückt, die bloße Annäherung der Schnittstelleneinheit und der Stabilisierungskomponente in die zweite Relativstellung sollte nicht für sich zu einer signifikanten Zunahme des Kontaktbereichs zwischen Gewebe und Gewebeanlagefläche sorgen. Dies kann beispielsweise dadurch gewährleistet werden, dass der Bewegungsweg zwischen erster und zweiter Relativstellung nur einen Bruchteil eines Millimeters beträgt.

Die Erfindung eignet sich insbesondere für einen automatisierten Verfahrensablauf, bei dem zumindest Teile des Ankopplungsvorgangs automatisiert ablaufen. Beispielsweise kann das Erreichen der ersten Relativstellung sensorisch erfasst werden und die Evakuierung der Saugkammer automatisch, insbesondere programmgesteuert in Antwort auf die Erfassung der ersten Relativstellung bewirkt werden. Alternativ oder zusätzlich kann die relative Annäherung der Schnittstelleneinheit an die Stabilisierungskomponente durch Betätigung einer motorischen Antriebseinrichtung bewirkt werden und der Betrieb der Antriebseinrichtung automatisch, insbesondere programmgesteuert in Antwort auf eine sensorische Erfassung der ersten Relativstellung gestoppt werden.

Gemäß einem weiteren Gesichtspunkt der Erfindung ist zur Lösung der eingangs genannten Aufgabe eine Vorrichtung wie in Anspruch 9 definiert zum Schneiden eines Gewebeteils eines Auges mittels fokussierter Laserstrahlung vorgesehen, wobei diese Vorrichtung insbesondere zur Durchführung des Verfahrens der vorstehend erläuterten Art geeignet ist. Als Kontaktglas sei hier jegliches transparente Kontaktelement verstanden, das zur formenden Anlage durch die Augenoberfläche dient. Der Wortbestandteil "Glas" soll nicht als Hinweis auf ein bestimmtes Glasmaterial des Kontaktglases verstanden werden; stattdessen soll er allein die Transparenz für die Laserstrahlung verdeutlichen. Auch wenn in der Praxis vielfach ein Glasmaterial für das Kontaktglas Verwendung finden kann, ist es ebenso vorstellbar, das Kontaktglas aus einem Kunststoffmaterial zu fertigen. Bei einer bevorzugten Ausführungsform ist das Kontaktglas als planparallele Applanationslinse ausgeführt, besitzt also auf seiner augenzugewandten Seite sowie auf seiner augenabgewandten Seite je eine plane Linsenfläche. Es ist im Rahmen der Erfindung freilich nicht grundsätzlich ausgeschlossen, das Kontaktglas mit nichtebenen Hauptseiten auszuführen.

Die Sensorik kann beispielsweise einen Näherungssensor umfassen, welcher z.B. als Hall-Sensor, als optischer Sensor oder als Reed-Schalter ausgebildet sein kann. Es ist auch vorstellbar, einen optischen Sensor nach Art einer Lichtschranke vorzusehen, um das Erreichen der vorgegebenen ersten Relativposition der Schnittstelleneinheit und der Saugringeinheit zu detektieren. Darüber hinaus ist es vorstellbar, einen Druck- oder Kraftsensor vorzusehen, welcher einen auf die Schnittstelleneinheit wirkenden Gegendruck vom Auge oder von der Saugringeinheit misst. Bei einer gewichtskompensierten Aufhängung der Bearbeitungsoptik einer die Laserstrahlung bereitstellenden Lasereinrichtung kann die Sensorik auch einen Schalter umfassen, welcher bei einer bestimmten Auslenkung der Optik aus einer Normallage schaltet, die sie erfährt, wenn die mit der Optik gekoppelte Schnittstelleneinheit auf die Augenoberfläche oder die Saugringeinheit trifft und so einen Gegendruck erfährt.

Die Saugringeinheit stellt ein Beispiel einer Stabilisierungskomponente im Sinne der Erfindung dar; sie stabilisiert und fixiert das Auge. Sie kann eine ringförmig umlaufende erste Dichtfläche bilden, mit welcher sie zur Abdichtung der ersten Saugkammer an die Augenoberfläche anlegbar ist, wobei die Evakuierung der ersten Saugkammer eine Anlage des Auges an das Kontaktglas in einem Bereich bewirkt, der angenähert der von der ersten Dichtfläche umschlossenen Fläche entspricht. Über den Durchmesser bzw. die Querschnittsgröße des von der ersten Dichtfläche umschlossenen Bereichs kann so die Größe des Kontaktbereichs zwischen Auge und Kontaktglas festgelegt werden.

Bei einer bevorzugten Ausführungsform trennt die erste Dichtfläche die erste Saugkammer von einer vollständig zwischen der Saugringeinheit und der Augenoberfläche begrenzten, mit der ersten Saugkammer unverbundenen und von dieser unabhängig evakuierbaren zweiten Saugkammer. Die zweite Saugkammer dient zur Ansaugung der Saugringeinheit an die Sclera des Auges. Beispielsweise kann die erste Dichtfläche am Innenrand eines zur Ringmitte hin abstehenden, radial oder schräg zur Radialen gerichteten Ringvorsprungs der Saugringeinheit gebildet sein, etwa von einer an diesem Ringvorsprung angebrachten Ringdichtung.

Bei ordnungsgemäß auf das Auge aufgesetzter Saugringeinheit kann in der ersten Relativstellung die erste Saugkammer vor ihrer Evakuierung vollständig an die augenzugewandte Unterseite des Kontaktglases grenzen. Bei einer solchen Ausführungsform hat in der ersten Relativstellung vor Evakuierung der ersten Saugkammer das Kontaktglas keinen Kontakt mit der Augenoberfläche. Wie bereits erläutert, ist es bei alternativen Ausführungsformen möglich, dass in der ersten Relativstellung bereits ein vergleichsweise kleinflächiger Kontakt zwischen der Augenoberfläche und dem Kontaktglas besteht.

Die erste Saugkammer kann axial in Richtung von dem Auge weg über das Kontaktglas hinausreichen.

Vorzugsweise bildet die Saugringeinheit einen sich in Richtung axial vom Auge weg öffnenden Einführtrichter, wobei die Schnittstelleneinheit einen Konusabschnitt zum axialen Einsetzen in den Einführtrichter aufweist. Das Zusammenwirken des Einführtrichters und des Konusabschnitts gestattet eine präzise Zentrierung der Schnittstelleneinheit relativ zu der Saugringeinheit. Zudem wird durch den Einführtrichter eine axiale Baulänge der Saugringeinheit erreicht, die gewährleistet, dass nicht unbeabsichtigtermaßen die Wimpern des Auges störend zwischen die Saugringeinheit und die Schnittstelleneinheit gelangen können.

Zweckmäßigerweise bilden der Einführtrichter und der Konusabschnitt zusammenwirkende zweite Dichtflächen zur Abdichtung der ersten Saugkammer. Dabei kann der Einführtrichter oder/und der Konusabschnitt eine umlaufende Nut besitzen, in welche ein Dichtring eingesetzt ist. Alternativ ist eine Dichtwirkung auch ohne gesondertes Dichtelement erzielbar, insbesondere wenn der Einführtrichter und der Konusabschnitt ausreichend glatte Flächen besitzen, die zur Anlage aneinander kommen und so die erste Saugkammer absichten können.

Die Schnittstelleneinheit kann einen zur Kopplung mit der Saugringeinheit ausgebildeten, das Kontaktglas tragenden Kontaktglashalter sowie einen von dem Kontaktglashalter gesonderten, fest, jedoch auswechselbar mit diesem verbindbaren Adapter umfassen, welcher mit Koppelformationen zur Ankopplung an eine Fokussieroptik einer die Laserstrahlung bereitstellenden Lasereinrichtung ausgeführt ist. Die Zweiteilung der Schnittstelleneinheit in einen Kontaktglashalter und einen Adapter ist insofern vorteilhaft, als nicht die gesamte Schnittstelleneinheit nach einer Operation wiederaufbereitet oder gar weggeworfen werden muss. Stattdessen kann der Adapter ohne weiteres wiederverwendet werden, gegebenenfalls nach vorheriger Sterilisierung. Der Kontaktglashalter mit dem Kontaktglas kann dagegen ein Wegwerfartikel sein. Der Kontaktglashalter und der Adapter können beispielsweise mit Gewindemitteln ausgeführt sein, welche eine Schraubverbindung des Kontaktglashalters und des Adapters gestatten. Für den Kontaktglashalter wird ein konusförmiger Hülsenkörper verwendet, dessen Hülsenmantel aus Vollmaterial besteht oder zur Gewichtsersparnis Durchbrechungen besitzt.

Die Erfindung wird nachfolgend anhand der beigefügten Zeichnungen weiter erläutert. Es stellen dar:
- Fig. 1a-1e: schematisch aufeinanderfolgende Phasen beim An- und Abkoppeln einer Schnittstelleneinheit an eine auf einem Auge sitzende Saugringeinheit gemäß einem Ausführungsbeispiel,
- Fig. 2: stark schematisiert ein Ausführungsbeispiel einer Lasereinrichtung zur Einbringung von Schnitten in die Hornhaut eines Auges,
- Fig. 3: einen Axiallängsschnitt durch ein realistisch gezeigtes Ausführungsbeispiel einer Schnittstelleneinheit und einer Saugringeinheit in einem gekoppelten Zustand,
- Fig. 4a und 4b: Perspektivansichten der Saugringeinheit gemäß Fig. 3, und zwar einmal im Ganzen und einmal hälftig aufgeschnitten, und
- Fig. 5: einen vergrößerten Ausschnitt aus Fig. 3.

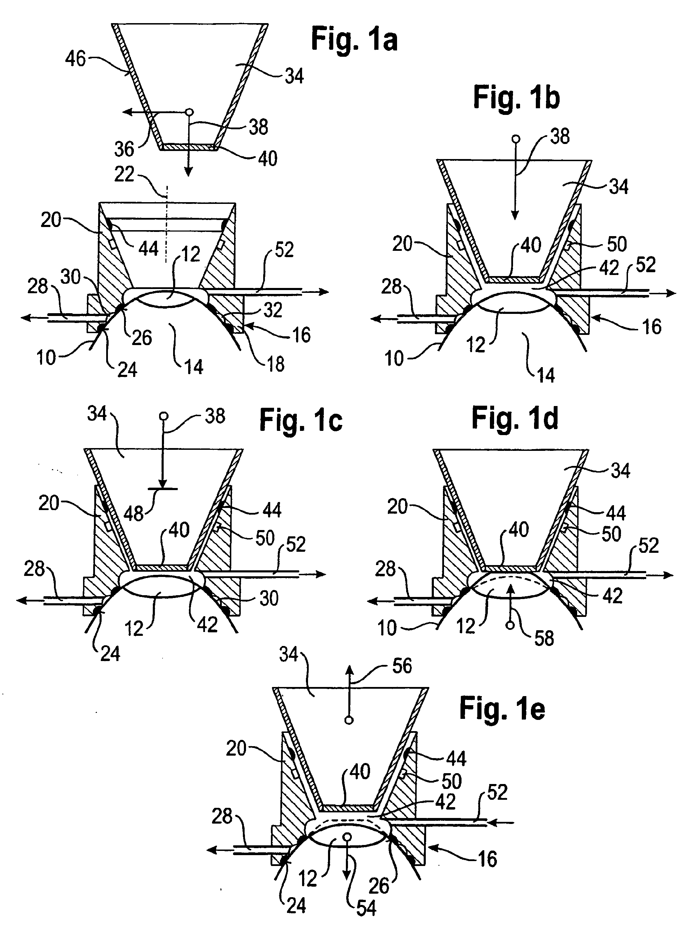

Bei dem in den Figuren 1a-1e gezeigten Ausführungsbeispiel ist das zu behandelnde (menschliche) Auge mit 10 bezeichnet. Die Kornea (Hornhaut) des Auges 10 ist bei 12 gezeigt, während die Sclera (Lederhaut) mit 14 bezeichnet ist.

In die Kornea 12 des Auges 10 sollen mittels gepulster Laserstrahlung mit Pulsdauern im Femtosekundenbereich ein oder mehrere Schnitte eingebracht werden. Die hierfür notwendige Laserstrahlung wird von einer nicht näher dargestellten Laserquelle bereitgestellt. Beispielsweise liegt die Wellenlänge der in das Auge 10 eingestrahlten Behandlungsstrahlung im niederen Infrarotbereich. Beispielsweise kann ein bei 1030 nm strahlender Yb-Laser verwendet werden.

Bevor mit der Laserbehandlung des Auges begonnen wird, ist zunächst das Auge 10 an die mit der Laserquelle ausgestattete Lasereinrichtung anzukoppeln, um den Strahlfokus präzise in z-Richtung in der Kornea positionieren zu können. Hierfür wird zunächst eine Saugringeinheit 16 in an sich bekannter Weise auf das Auge 10 aufgesetzt und durch Unterdruck an dem Auge 10 fixiert. Die Saugringeinheit 16 stabilisiert und fixiert das Auge 10. Sie weist einen den eigentlichen Saugring bildenden Unterteil 18, einen an den Unterteil 18 anschließenden, einstückig mit diesem hergestellten Einführtrichter 20 sowie eine Ringachse 22 auf. Der Unterteil 18 bildet zwei jeweils zur Anlage an der Sclera 14 bestimmte, ringförmig umlaufende Dichtflächen 24, 26, welche zwischen sich eine mit einem Evakuierungskanal 28 verbundene, ringförmig umlaufende Saugkammer 30 begrenzen. Die Dichtflächen 24, 26 können beispielsweise jeweils von einem gesonderten, am Untereil 18 angebrachten Dichtelement gebildet sein. Zur Bildung der Saugkammer 30 ist am augenzugewandten Innenumfangsmantel des Unterteils 18 eine entsprechende Ringnut - bezeichnet mit 32 - gebildet. Die Saugkammer 30 ist ausschließlich zwischen der Saugringeinheit 16 und der Sclera 14 begrenzt. Durch Evakuierung der Saugkammer 30 saugt sich die Saugringeinheit 16 am Auge 10 fest. Hierzu wird der Evakuierungskanal 28 mit einer nicht näher dargestellten Unterdruckquelle in Form einer Evakuierungspumpe verbunden.

Mechanisches Gegenstück zu der solchermaßen auf dem Auge 10 fixierten Saugringeinheit 16 ist eine allgemein mit 34 bezeichnete Schnittstelleneinheit, welche in nicht näher dargestellter Weise fest, jedoch lösbar mit einer Fokussieroptik der erwähnten Lasereinrichtung koppelbar ist. Die Schnittstelleneinheit 34 ist zusammen mit der Fokussieroptik entlang einer durch einen Horizontalpfeil 36 angegebenen Horizontalrichtung und einer durch einen Vertikalpfeil 38 angedeuteten Vertikalrichtung relativ zu dem Patienten und der an ihm befestigten Saugringeinheit 16 verfahrbar. Die Verfahrbarkeit der Schnittstelleneinheit 34 kann zumindest teilweise motorisch bewirkt sein, beispielsweise mittels eines elektromotorischen Antriebs. Auch eine wenigstens teilweise händische Bewegbarkeit der Schnittstelleneinheit 34 relativ zu der Saugringeinheit 16 ist vorstellbar.

Insgesamt ist die Schnittstelleneinheit 34 kegelförmig ausgebildet, wobei sie an ihrem breiteren Kegelende (in den Figuren 1a-1e obenliegend) zur Kopplung mit der Fokussieroptik ausgebildet ist und an ihrem schmäleren Kegelende ein im gezeigten Beispielfall als planparallele Applanationslinse ausgebildetes Kontaktglas 40 trägt.

In einer ersten Phase des Ankoppelvorgangs des Auges 10 an die Lasereinrichtung wird die Schnittstelleneinheit 34 relativ zu der Saugringeinheit 16 in Pfeilrichtung 36 in eine Position bewegt, in der sie axial über dem Einführtrichter 20 steht, so dass die Schnittstelleneinheit 34 anschließend durch axiales Absenken in den Einführtrichter 20 eintauchen kann. Die Phase des Eintauchens der Schnittstelleneinheit 34 in den Einführtrichter 20 der Saugringeinheit 16 ist in Fig. 1b dargestellt. Beim Absenken der Schnittstelleneinheit 34 nähert sich die Applanationslinse 40 dem Auge 10; gleichzeitig wird der radiale Luftspalt zwischen dem Einführtrichter 20 und der Schnittstelleneinheit 34 kleiner. Der konusförmig verlaufende Innenumfangsmantel des Einführtrichters 20 und der gleichermaßen konusförmig verlaufende Außenumfangsmantel der Schnittstelleneinheit 34 tragen oder bilden jeweils eine Dichtfläche. Diese zusammenwirkenden Dichtflächen gelangen im Zuge des weiteren Ankoppelvorgangs zur Anlage aneinander und dichten in diesem Zustand eine weitere Saugkammer 42 ab. Die weitere Saugkammer 42 ist zwischen der Saugringeinheit 16, der Schnittstelleneinheit 34 und der Oberfläche des Auges 10 gebildet. Als untere Begrenzung der Saugkammer 42 dient die Dichtfläche 26, die dementsprechend beide Saugkammern 30, 42 zugleich abdichtet. Es versteht sich, dass alternativ eine von der Dichtfläche 26 gesonderte, weitere Ringdichtfläche an der Saugringeinheit 16 gebildet sein kann, welche der Abdichtung der Saugkammer 42 dient.

Die zwischen der Saugringeinheit 16 und der Schnittstelleneinheit 34 wirksamen Dichtflächen sind im gezeigten Beispielfall von einer an dem Einführtrichter 20 angebrachten Ringdichtung 44 und dem dieser Ringdichtung 44 im eingetauchten Zustand gegenüberliegenden Teil des Außenumfangsmantels der Schnittstelleneinheit 34 gebildet. Dieser als Dichtfläche wirkende Teil des Außenumfangsmantels der Schnittstelleneinheit 34 ist in Figur 1a mit 46 bezeichnet. Die Ringdichtung 44 kann beispielsweise eine Lippendichtung oder ein O-Ring sein. Es versteht sich, dass alternativ eine solche Ringdichtung an der Schnittstelleneinheit 34 vorgesehen sein kann. Es ist darüber hinaus vorstellbar, auf ein gesondertes Dichtelement zu verzichten, sofern die Außenoberfläche der Schnittstelleneinheit 34 und die Innenoberfläche des Einführtrichters 20 hinreichend glatt sind und hinreichend eng aneinander zu liegen kommen.

Die Absenkung der Schnittstelleneinheit 34 in Pfeilrichtung 38 (entsprechend der Axialrichtung der Saugringeinheit 16) stoppt an einer vorgegebenen axialen Relativposition, in der die Schnittstelleneinheit 34 noch gewissen axialen Abstand von der Saugringeinheit 16 hat, also noch nicht maximal tief in den Einführtrichter 20 eingetaucht ist. Der Stopp der Absenkbewegung ist in Fig. 1c durch einen Querstrich 48 schematisch angedeutet. In der Stoppposition der Schnittstelleneinheit 34 besteht einerseits noch ein radialer Spalt zwischen dem Einführtrichter 20 und der Schnittstelleneinheit 34, andererseits ist noch keine signifikante Einebnung des Auges durch die Applanationsplatte 40 erfolgt. Beispielsweise beträgt die Größe des Luftspalts zwischen Einführtrichter 20 und Schnittstelleneinheit 34 bei Erreichen der Stoppposition weniger als 0,5 mm bis hin zu nicht mehr als 0,1 mm.

Im gezeigten Beispielfall der Fig. 1c ist in der Stoppposition der Schnittstelleneinheit 34 überhaupt kein Kontakt zwischen der Applanationsplatte 40 und dem Auge 10 gegeben; stattdessen befindet sich die Applanationsplatte 40 in geringem axialen Abstand über der Kornea 12 des Auges 10. Es kann alternativ in der Stoppposition aber auch ein geringfügiger Kontakt zwischen der Applanationsplatte 40 und dem Auge bestehen; die Fläche des Kontaktbereichs ist dann freilich beträchtlich kleiner als der für die spätere Behandlung erforderliche Applanationsbereich. Beispielsweise kann der angestrebte Einebnungsbereich des Auges einen Durchmesser zwischen etwa 10 und 11 mm aufweisen. Sofern in der Stoppposition gemäß Fig. 1c überhaupt ein Kontakt zwischen der Applanationsplatte 40 und dem Auge 10 vorhanden ist, wird der Durchmesser des Kontaktbereichs dagegen vorzugsweise nur höchstens einige wenige Millimeter betragen, beispielsweise nur etwa 2 oder 3 mm. In jedem Fall ist ein in der Stoppposition etwa bestehender Kontakt zwischen Applanationsplatte 40 und Auge 10 so schwach, dass durch den Kontakt keine signifikante Erhöhung des Augeninnendrucks bewirkt wird.

Die Stoppposition der Schnittstelleneinheit 40 wird im gezeigten Beispielfall durch ein an der Saugringeinheit 16, genauer am Einführtrichter 20 angeordnetes Sensorelement 50 detektiert, bei dem es sich beispielsweise um einen als Näherungssensor ausgebildeten Hall-Sensor handeln kann. Das Sensorelement 50 ist geeignet positioniert, um dann ein entsprechendes Sensorsignal abzugeben, wenn die Schnittstelleneinheit 34 die vorgegebene Stoppposition erreicht.

In der Stoppposition erstreckt sich die Saugkammer 42 in den zwischen dem Einführtrichter 20 und der Schnittstelleneinheit 34 noch bestehenden Ringspalt hinein. Das Dichtelement 44 kann bereits in Dichtanlage am konischen Außenumfangsmantel der Schnittstelleneinheit 34 sein. Es ist aber auch möglich, dass in der Stoppposition noch keine vollständige Abdichtung der Saugkammer 42 gegeben ist. Je nachdem, ob in der Stoppposition bereits ein Kontakt zwischen der Applanationsplatte 40 und dem Auge 10 vorhanden ist oder nicht, erstreckt sich die Saugkammer 42 entweder vollständig zwischen der Applanationsplatte 40 und dem Auge 10 hindurch oder umschließt den bestehenden Kontaktbereich.

Die Saugkammer 42 ist mit einem weiteren Evakuierungskanal 52 verbunden, der wie der Evakuierungskanal 28 an der Saugringeinheit 16 ausgebildet ist und ebenfalls mit einer als Unterdruckquelle dienenden Evakuierungspumpe (nicht näher dargestellt) verbindbar ist. Über die beiden Evakuierungskanäle 28, 52 können die beiden Saugkammern 30, 42 unabhängig voneinander evakuiert werden. Hierzu können zwei gesonderte, unabhängig voneinander betreibbare Evakuierungspumpen vorgesehen sein. Es ist alternativ vorstellbar, eine einzige Evakuierungspumpe vorzusehen und durch geeignet steuerbare Ventilmittel die beiden Saugkammern 30, 42 individuell mit Unterdruck zu versorgen.

Ausgehend von der Stoppposition gemäß Fig. 1c wird als nächste Aktion die Saugkammer 42 evakuiert. Der zunehmende Unterdruck in der Saugkammer 42 bewirkt eine Ansaugung der Augenoberfläche an die (augenzugewandte) Unterseite der Applanationsplatte 40. Gleichzeitig wird die Schnittstelleneinheit 34 durch die Saugwirkung tiefer in den Einführtrichter 20 der Saugringeinheit 16 hineingezogen, bis sie letztendlich eine maximal tiefe Eintauchposition erreicht. Dieses weitere Absinken der Schnittstelleneinheit 34 wird allein durch die Saugwirkung des in der Saugkammer 42 herrschenden Unterdrucks bewirkt; eine etwaige motorische oder händische Kraftunterstützung findet in dieser Phase des Ankoppelvorgangs nicht mehr statt. Das Maß dieser weiteren Absenkbewegung der Schnittstelleneinheit 34 ist vergleichsweise gering; beispielsweise wird die Schnittstelleneinheit 34 saugkraftbedingt nur einige Zehntel Millimeter tiefer in den Einführtrichter 20 hineingesogen.

Die Stärke des in der Saugkammer 42 erzeugten Unterdrucks ist ausreichend hoch gewählt, um das Auge 10 in dem für die Behandlung gewünschten Bereich einzuebnen, d.h. durch Ansaugung an die Platte 40 zu applanieren. Dabei ist der durch das Hineinziehen der Schnittstelleneinheit 40 in den Einführtrichter 20 bewirkte Zuwachs an Kontakt in jedem Fall klein und vorzugsweise sogar vernachlässigbar gegenüber der infolge der Ansaugung der Kornea bewirkten Kontaktbereichsvergrößerung. Um beispielhafte Zahlenwerte zu nennen, so kann in der Saugkammer 42 beispielsweise ein Unterdruck zwischen 200 und 600 mm Hg erzeugt werden, vorzugsweise zwischen 400 und 500 mm Hg.

Der applanierte Zustand, in dem die Saugkammer 42 auf das gewünschte Niveau evakuiert ist, ist in Fig. 1d gezeigt. In diesem Zustand kann durch Einstrahlung von Laserstrahlung von oben her durch die Applanationsplatte 40 die gewünschte Behandlung des Auges 10 erfolgen, beispielsweise die Erzeugung eines Flaps im Rahmen einer Fs-LASIK-Behandlung.

Sollte es in dem Zustand gemäß Fig. 1d Probleme geben, etwa weil der Patient panisch reagiert, kann durch Belüftung der Saugkammer 42 eine rasche Abkopplung der Schnittstelleneinheit 34 von der Saugringeinheit 16 erreicht werden. Diese Situation ist in Fig. 1e dargestellt. Ein nach unten gerichteter Pfeil 54 verdeutlicht die augenblickliche Loslösung des Auges 10 von der Applanationsplatte 40. Wegen der fehlenden Saugwirkung kann dann auch die Schnittstelleneinheit 34 axial nach oben aus dem Einführtrichter 20 entweichen. Dies ist durch einen aufwärts gerichteten Pfeil 56 veranschaulicht.

Im Vergleich zu Fig. 1e ist in Fig. 1d die auf die Augenoberfläche ausgeübte Saugwirkung durch einen zur Applanationsplatte 40 hin gerichteten Aufwärtspfeil 58 veranschaulicht.

Bei einem Prozess, wie er oben erläutert wurde, ist es möglich, eine durch die Ankopplung der Schnittstelleneinheit 34 an die Saugringeinheit 16, d.h. durch die Einebnung der Kornea bewirkte Erhöhung des Augeninnendrucks nicht nur absolut klein zu halten sondern auch klein im Vergleich zu einer Druckerhöhung infolge des Ansaugens der Saugringeinheit 16 an die Sclera. Um ein zahlenmäßiges Beispiel zu geben, so kann die Fixierung der Saugringeinheit 16 am Auge bereits eine Erhöhung des Augeninnendrucks um etwa 60 bis 100 mm Hg bewirken. Zum Vergleich, übliche Innendrücke des menschlichen Auges ohne Berührung liegen in der Regel zwischen etwa 15 und 20 mm Hg. Liegt in der Stoppposition der Schnittstelleneinheit 34 vor Beginn der Evakuierung der Saugkammer 42 schon ein Kontakt zwischen Auge und Applanationsplatte 40 vor, so ist der hierdurch bewirkte Druckanstieg im Auge vorzugsweise kleiner als 10 mm Hg. Ein solch geringer Druckanstieg kann ohne weiteres mit einem Kontakt mit 2 bis 3 mm Durchmesser gewährleistet werden. In jedem Fall sollte ein in der Stoppposition vor Beginn der Evakuierung bestehender Kontakt vorzugsweise nicht zu einem Druckanstieg um mehr als 20 mm Hg führen. Durch das anschließende Evakuieren der Saugkammer 42 und die Ansaugung des Auges an die Applanationsplatte kann ein weiterer leichter Druckanstieg im Auge entstehen, der jedoch abhängig vom Unterdruck in der Saugkammer 42 in der Regel nicht mehr als ca. 20 mm Hg betragen sollte.

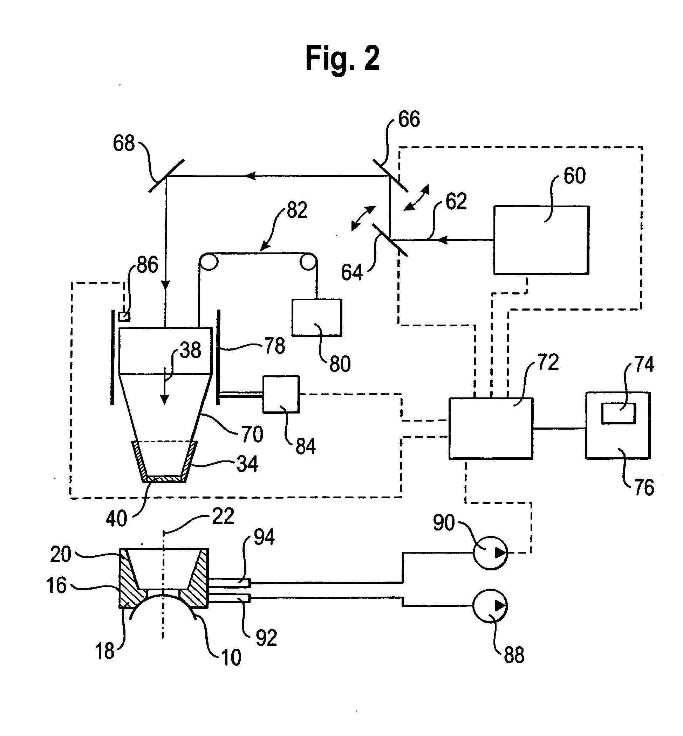

Fig. 2 zeigt schematisch Komponenten einer Lasereinrichtung, mit der das zuvor anhand der Figuren 1a-1e beschriebene Verfahren durchgeführt werden kann. Gleiche oder gleichwirkende Komponenten wie in den Figuren 1a-1e sind dabei mit gleichen Bezugszeichen bezeichnet. Zur Vermeidung von Wiederholungen wird auf die vorangehenden Ausführungen zu diesen Komponenten verwiesen.

Die Lasereinrichtung gemäß Fig. 2 umfasst eine Laserquelle 60 für gepulste Laserstrahlung mit Pulsdauern im Femtosekundenbereich. Der von der Laserquelle 60 abgegebene Laserstrahl - mit 62 bezeichnet - gelangt über eine hier von zwei steuerbaren Ablenkspiegeln 64, 66 gebildete Ablenkeinrichtung (Scanner) zu einem Umlenkspiegel 68, von welchem der Laserstrahl 62 in eine Fokussieroptik 70 gelangt. Am distalen, d.h. augennahen Ende der Fokussieroptik 70 ist die Schnittstelleneinheit 34 lösbar angekoppelt. Die Ablenkspiegel 64, 66 sind jeweils kippbar angeordnet und gestatten eine Ablenkung des Laserstrahls 62 in einer zur Strahllängsrichtung (z-Richtung) normalen x-y-Ebene. Sie werden von einer elektronischen Steuereinrichtung 72 nach Maßgabe eines durch Form und Lage des gewünschten Schnitts gegebenen Schnittprofils gesteuert. Das Schnittprofil ist in einem Steuerprogramm 74 verkörpert, welches in einem für die Steuereinrichtung 72 zugänglichen Speicher 76 abgelegt ist. Zur z-Verstellung des Strahlfokus kann entweder die Fokussieroptik 70 oder zumindest eine darin enthaltene Linse in Strahllängsrichtung unter der Steuerung der Steuereinrichtung 72 justierbar sein. Alternativ ist es möglich, eine Linse einer in Fig. 2 nicht näher dargestellten, zwischen der Laserquelle 60 und den Ablenkspiegeln 64, 66 angeordneten Strahlaufweitungsoptik in Strahllängsrichtung verstellbar anzuordnen, insbesondere eine eingangsseitige Zerstreuungslinse einer solchen Strahlaufweitungsoptik.

Die Fokussieroptik 70 ist an einer Halterung 78 gewichtskompensiert aufgehängt. Die Halterung 78 ist in Fig. 2 stark schematisch durch zwei beidseits der Fokussieroptik 70 gezeichnete senkrechte Striche angedeutet. Die Gewichtskompensation der Fokussieroptik 70 ist schematisch durch ein Gegengewicht 80 angedeutet, das über eine Seil-/Rollen-Anordnung 82 mit der Fokussieroptik 70 verbunden ist und eine deren Gewichtskraft kompensierende Gegenkraft auf die Fokussieroptik 70 ausübt. Eine Seil-/Rollen-Anordnung ist selbstverständlich nur ein Beispiel für die Anbindung eines Gegengewichts an die Fokussieroptik. Alternativ könnte beispielsweise ein Hebelsystem verwendet werden. Eine weitere mögliche Ausgestaltung ist in US 5,336,215 gezeigt, wo ein Federsystem zur Aufhängung einer Fokussieroptik verwendet wird.

Die Fokussieroptik 70 ist zusammen mit der Halterung 78 durch eine motorische, vorzugsweise elektromotorische Antriebseinheit 84 in vertikaler Richtung in den Einführtrichter 20 der auf dem Auge 10 sitzenden Saugringeinheit 16 absenkbar, wie durch den Richtungspfeil 38 angedeutet. Dabei ist die Fokussieroptik 70 nicht starr mit der Halterung 78 verbunden, sondern besitzt gegenüber der Halterung 78 eine gewisse Auslenkbarkeit entgegen der Absenkrichtung 38 nach oben. Wegen der Gewichtskompensation der Fokussieroptik 70 ist eine Auslenkung derselben gegenüber der Halterung bereits durch eine äußerst geringe Krafteinwirkung möglich. Der Moment, in dem die Applanationsplatte 40 in Kontakt mit dem Auge gelangt und einen Gegendruck vom Auge erfährt, kann deshalb schon zu einer Auslenkung der Fokussieroptik 70 relativ zu der Halterung 78 führen. Diese Auslenkung wird mittels eines relativ zu der Halterung 78 ortsfest angebrachten Endschalters 86 (alternativ z.B. Gegenkraftschalter) detektiert, der sein Schaltsignal an die Steuereinrichtung 72 liefert. Das Schalten des Endschalters 86 signalisiert der Steuereinrichtung 72 somit das Erreichen der vorgegebenen Relativstellung zwischen Schnittstelleneinheit 34 und Saugringeinheit 16 (z.B. Berühren des Einführtrichters 20 durch die Schnittstelleneinheit 34), in der die weitere motorische Absenkbewegung der Fokussieroptik 70 zu stoppen ist. Dementsprechend steuert die Steuereinrichtung 72 bei Erhalt des Schaltsignals von dem Endschalter 86 die Antriebseinheit 84 im Sinne eines Betriebsstopps. Das vorherige Absenken der Fokussieroptik 70 durch die Antriebseinheit 84 kann ebenfalls von der Steuereinrichtung 72 nach Maßgabe des Steuerprogramms 74 gesteuert sein; alternativ ist es vorstellbar, dass der Bediener die Absenkbewegung mittels eines an die Steuereinrichtung 72 angeschlossenen Steuerknüppels händisch initialisiert, wobei bei Erreichen der erwähnten vorgegebenen Relativposition zwischen Schnittstelleneinheit 34 und Saugringeinheit 16 die Steuereinrichtung 72 den Vorrang des Steuerknüppels aufhebt und selbsttätig den Betrieb der Antriebseinheit 84 stoppt.

Wegen des Vorhandenseins des Endschalters 86 ist in diesem Fall ein an den Komponenten 16 oder/und 34 angebrachter Sensor verzichtbar.

Die Lasereinrichtung gemäß Fig. 2 umfasst darüber hinaus zwei Evakuierungspumpen 88, 90, welche über geeignete Schlauchleitungen an je einen an der Saugringeinheit 16 ausgebildeten Anschlussstutzen 92 bzw. 94 angeschlossen sind. In den Anschlussstutzen 92 mündet der Evakuierungskanal 28 gemäß den Fig. 1a-1e; die Evakuierungspumpe 88 dient somit zur Evakuierung der Saugkammer 30. Dagegen mündet in den Anschlussstutzen 94 der Evakuierungskanal 52, weswegen die Evakuierungspumpe 90 zur Evakuierung der Saugkammer 42 der Figuren 1a-1e dient. Zumindest die Evakuierungspumpe 90 ist im dargestellten Beispielfall gemäß Fig. 2 durch die Steuereinrichtung 72 steuerbar, nämlich in Antwort auf das Schalten des Endschalters 86. Das heißt, es erfolgt ein automatisches Einschalten der Pumpe 90, sobald die Schnittstelleneinheit 34 ihre vorgegebene Relativposition gegenüber der Saugringeinheit 16 erreicht hat. Das Schalten des Endschalters 86 kann im übrigen nicht nur durch ein Auslenken der Fokussieroptik 70 infolge eines Kontakts mit dem Auge ausgelöst werden. Beispielsweise kann der hierfür erforderliche Gegendruck auch von einer an dem Einführtrichter 20 oder der Schnittstelleneinheit 34 angebrachten Ringdichtung (z.B. Lippendichtung) erzeugt werden, etwa der in den Fig. 1a-1e gezeigten Ringdichtung 44.

Im Rahmen eines vollautomatischen Betriebs der Lasereinrichtung kann auch die Evakuierungspumpe 88 von der Steuereinrichtung 72 steuerbar sein. Es ist aber ebenso vorstellbar, dass zumindest das Festsaugen der Saugringeinheit 16 an das Auge 10 durch manuelle Betätigung der Evakuierungspumpe 88 seitens des operierenden Arztes bewirkt wird. Eine solche manuelle Ein- und Abschaltbarkeit ist im übrigen auch für die Pumpe 90 nicht ausgeschlossen. Insbesondere für den Fall von Komplikationen sollte zumindest eine manuelle Abschaltbarkeit der Evakuierungspumpe 90 für den bedienenden Arzt möglich sein.

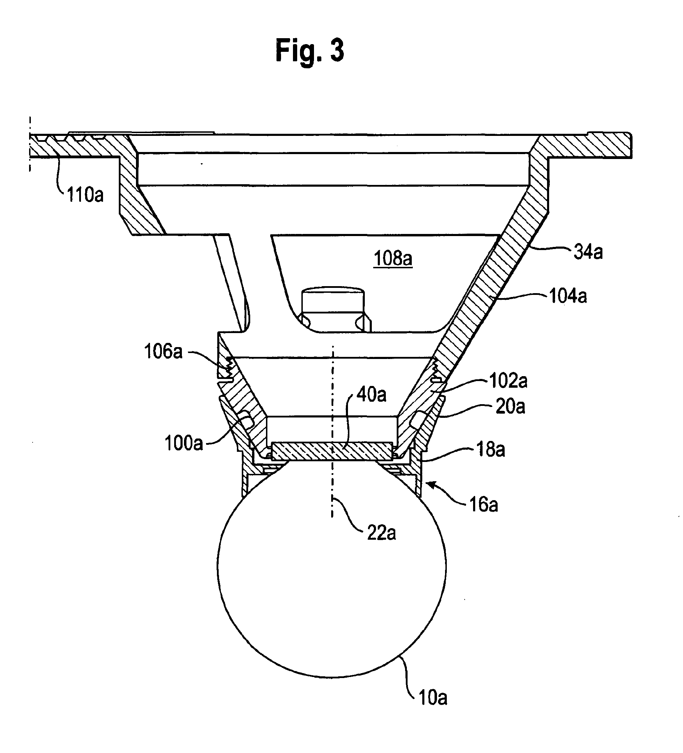

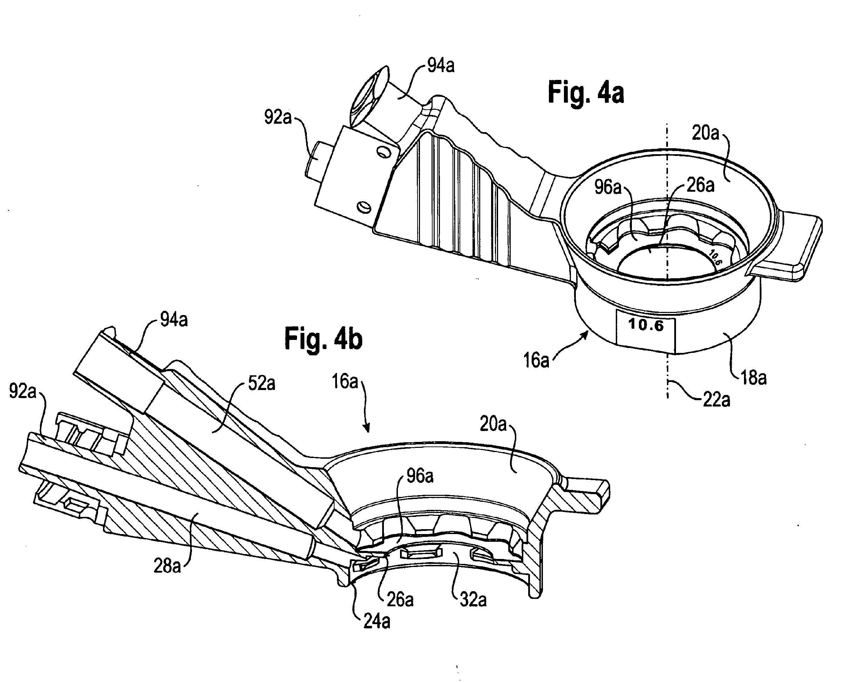

Bei der Erläuterung des Ausführungsbeispiels der Fig. 3-5 wird erneut auf gleiche Bezugszeichen wie zuvor zurückgegriffen, soweit es sich um gleiche oder gleichwirkende Komponenten wie in den vorangehenden Ausführungsbeispielen handelt. Zur Unterscheidung ist diesmal jedoch ein Kleinbuchstabe an das betreffende Bezugszeichen angehängt. Soweit sich nachstehend nichts anderes ergibt, wird für eine Erläuterung der erwähnten gleichen oder gleichwirkenden Komponenten auf das vorstehend Gesagte verwiesen.

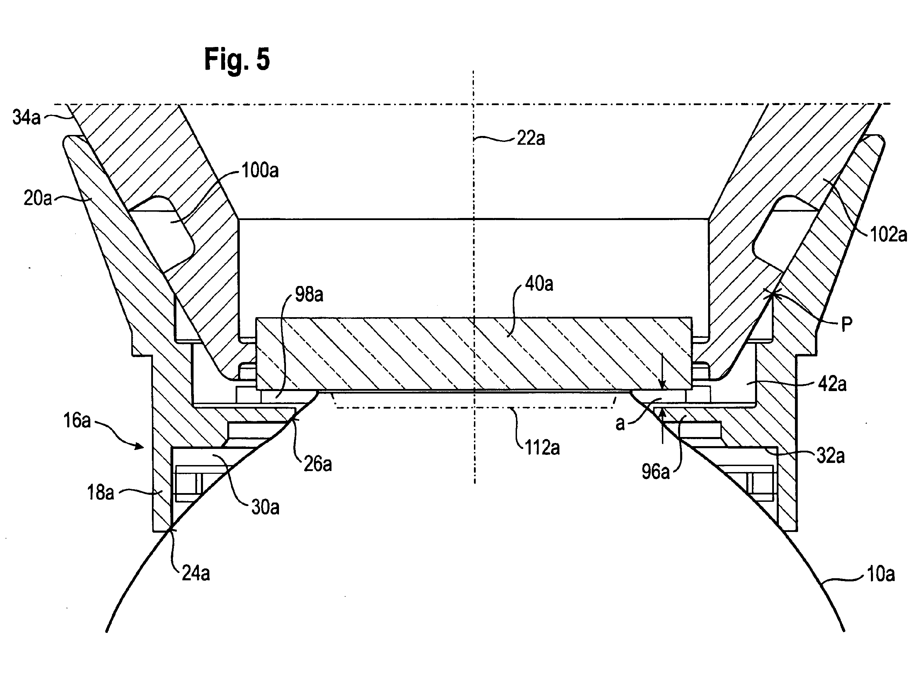

Der Unterteil 18a der Saugringeinheit 16a besitzt bei dem Ausführungsbeispiel der Fig. 3-5 eine radial zum Ringzentrum hin abstehende, ringscheibenförmige Dicht- und Trennplatte 96a, welche an ihrem Innenrand unmittelbar die Dichtfläche 26a bildet oder einen die Dichtfläche 26a bildenden Dichtring trägt. Die Dicht- und Trennplatte 96a trennt die beiden Saugkammern 30a, 42a voneinander. Man erkennt, dass die Applanationsplatte 40a radial über die Dicht- und Trennplatte 96a hinausreicht und dass sich die Saugkammer 42a in den Bereich zwischen der Applanationsplatte 40a und der Dicht- und Trennplatte 96a hinein erstreckt. In dem Zustand gemäß den Figuren 3 und 5, in dem die Schnittstelleneinheit 34a ordnungsgemäß an die Saugringeinheit 16a angekoppelt ist und die Saugkammer 42a evakuiert ist, beträgt der axiale Abstand zwischen der augenzugewandten Unterseite der Applanationsplatte 40a und der Oberseite der Dicht- und Trennplatte 96a beispielsweise etwa 0,7 mm. Insgesamt hat sich für diesen Abstand ein Maß als zweckmäßig erwiesen, das nicht kleiner als 0,4 mm sein sollte, um unerwünschte Druckeinwirkungen der Applanationsplatte auf das Auge zu vermeiden, zugleich aber nicht größer als 1,2 mm, um das Auge in einem hinreichend großen Kontaktbereich an die Applanationsplatte 40a ansaugen und dadurch einebnen zu können. Zur Verdeutlichung ist der genannte axiale Abstand in Fig. 5 mit a identifiziert. Je nach Ausgestaltung der Applanationsplatte 40a (sie könnte auch stufig ausgeführt sein anstatt vollständig eben) kann der genannte Abstand zumindest im Randbereich der Platte 40a unter Umständen auch bis zu 0,1 mm klein sein.

Wie bereits erläutert, kann die Evakuierung der Saugkammer 42a dazu führen, dass die Schnittstelleneinheit 34a noch um ein vergleichsweise geringes axiales Maß tiefer in den Einführtrichter 20a der Saugringeinheit 16a hineingesogen wird. Zu Beginn der Evakuierung ist deshalb der Abstand a um dieses Maß größer als im evakuierten Zustand gemäß den Fig. 3 und 5.

Man erkennt in Fig. 5 ferner, dass sich die Saugkammer 42a seitlich außerhalb an dem unteren Kegelende der Schnittstelleneinheit 34a vorbei nach axial oben bis in einen Bereich erstreckt, der axial oberhalb der Applanationsplatte 40a liegt. Dieser Axialpunkt ist in Fig. 5 mit P bezeichnet. Jenseits des Punkts P, d.h. in Richtung zu größeren Öffnungsweiten des Einführtrichters 20a hin, ist in den konischen Außenumfangsmantel der Schnittstelleneinheit 34a eine umlaufende Ringnut 100a eingeformt, welche zur Aufnahme eines hier nicht näher dargestellten Dichtelements dient, das mit der inneren Trichterfläche des Einführtrichters 20a zur Abdichtung der Saugkammer 42a zusammenwirkt.

In Fig. 3 ist ferner zu erkennen, dass die Schnittstelleneinheit 34a zweiteilig ausgeführt sein kann und eine die Applanationsplatte 40a haltende Konushülse 102a sowie einen Adapterkegel 104a umfasst, der lösbar mit der Konushülse 102a verbunden ist, beispielsweise durch eine Schraubverbindung, wie bei 106a angedeutet. Allein die Konushülse 102a taucht in den Einführtrichter 20a ein; der Adapterkegel 104a bleibt stets axial außerhalb der Saugringeinheit 16a. Im gezeigten Beispielfall ist die Wand des Adapterkegels 104a von mehreren Durchbrechungen 108a durchsetzt, um Gewicht zu sparen. Die Konushülse 102a ist dagegen als Vollmantelkörper ausgebildet. Am proximalen, d.h. augenfernen Ende ist der Adapterkegel 104a zur lösbaren Kopplung (beispielsweise in Form einer axialen Klemmkopplung) mit der Fokussieroptik der Lasereinrichtung ausgeführt. Im gezeigten Beispielfall weist er hierzu einen als Klemmflansch dienenden, radial abstehenden Ringbund 110a auf.

Es versteht sich, dass alternativ eine einteilige oder sogar mehr als zweiteilige Ausgestaltung der Schnittstelleneinheit denkbar ist.

Eine strichpunktierte Linie 112a in Fig. 5 veranschaulicht eine beispielhafte Schnittform, wie sie in der Kornea des Auges 10a mittels geeignet in Raum und Zeit gesteuerter Laserpulse angebracht werden kann. Es versteht sich, dass eine Vielzahl anderer Schnittformen abhängig von der gewünschten Behandlungsart möglich sind.

## Patentansprüche

1. Verfahren zum Ankoppeln einer mechanischen Schnittstelleneinheit (34) einer Lasereinrichtung an eine an einem Augengewebe durch Saugkraft gehaltene Saugringeinheit (16), wobei die Saugringeinheit eine Ringachse (22) aufweist und einen sich in Richtung axial vom Auge weg öffnenden Einführtrichter bildet, wobei die Schnittstelleneinheit (34) einen Konusabschnitt zum axialen Einsetzen in den Einführtrichter aufweist, umfassend die Schritte:
- relatives Annähern der Schnittstelleneinheit (34) an die Saugringeinheit (16) bis in eine erste Relativstellung, in welcher die Schnittstelleneinheit (34) mit ihrem Konusabschnitt in den Einführtrichter der Saugringeinheit (16) eingetaucht ist,
- in der ersten Relativstellung Evakuieren einer zwischen der Schnittstelleneinheit (34), der Saugringeinheit (16) und der Oberfläche des Gewebes gebildeten Saugkammer (42), um dadurch einen Kontakt zwischen dem Gewebe und einer Gewebeanlagefläche der Schnittstelleneinheit (34) herzustellten oder einen Bereich bestehenden Kontakts zu vergrößern,
wobei die Evakuierung der Saugkammer (42) eine relative Bewegung der Schnittstelleneinheit (34) und der Saugringeinheit (16) in eine zweite Relativstellung bewirkt, in der sie stärker einander angenähert sind als in der ersten Relativstellung.

2. Verfahren nach Anspruch 1, **dadurch gekennzeichnet, dass** die Evakuierung der Saugkammer (42) eine Vergrößerung des Kontaktbereichs zwischen dem Gewebe und der Gewebeanlagefläche auf mindestens das Eineinhalbfache, vorzugsweise auf mindestens das Doppelte, insbesondere um ein Mehrfaches bewirkt.

3. Verfahren nach Anspruch 1 oder 2, **dadurch gekennzeichnet, dass** der Kontaktbereich zwischen dem Gewebe und der Gewebeanlagefläche näherungsweise kreisscheibenförmig ist und die Evakuierung der Saugkammer (42) eine Vergrößerung des Durchmessers des Kontaktbereichs um mindestens 30 Prozent, vorzugsweise um mindestens 50 Prozent, noch bevorzugter um mindestens 70 Prozent und höchstbevorzugt um mindestens 90 Prozent bewirkt.

4. Verfahren nach einem der vorhergehenden Ansprüche, **dadurch gekennzeichnet, dass** die relative Annäherung der Schnittstelleneinheit (34) an die Saugringeinheit (16) in einer Richtung (38) erfolgt, längs der über die erste Relativstellung hinaus eine weitere relative Annäherung der Schnittstelleneinheit (34) und der Saugringeinheit (16) insbesondere bis in einen Zustand wechselseitigen Anschlags möglich ist.

5. Verfahren nach einem der vorhergehenden Ansprüche, **dadurch gekennzeichnet, dass** der relative Bewegungsweg der Schnittstelleneinheit (34) und der Saugringeinheit (16) zwischen der ersten und der zweiten Relativstellung nicht mehr als 1 mm beträgt.

6. Verfahren nach einem der vorhergehenden Ansprüche, **dadurch gekennzeichnet, dass** ein auf die relative Annäherung der Schnittstelleneinheit (34) und der Saugringeinheit (16) von der ersten in die zweite Relativstellung zurückzuführender Anteil an der Vergrößerung des Kontaktbereichs zwischen dem Gewebe und der Gewebeanlagefläche klein, insbesondere vernachlässigbar klein im Vergleich zu einem auf das Ansaugen des Gewebes an die Gewebeanlagefläche zurückzuführenden Anteil ist.

7. Verfahren nach einem der vorhergehenden Ansprüche, **dadurch gekennzeichnet, dass** das Erreichen der ersten Relativstellung sensorisch erfasst wird und die Evakuierung der Saugkammer (42) automatisch, insbesondere programmgesteuert in Antwort auf die Erfassung der ersten Relativstellung bewirkt wird.

8. Verfahren nach einem der vorhergehenden Ansprüche, **dadurch gekennzeichnet, dass** die relative Annäherung der Schnittstelleneinheit (34) an die Saugringeinheit (16) durch Betätigung einer motorischen Antriebseinrichtung (84) bewirkt wird und dass der Betrieb der Antriebseinrichtung automatisch, insbesondere programmgesteuert in Antwort auf eine sensorische Erfassung der ersten Relativstellung gestoppt wird.

9. Vorrichtung zum Schneiden eines Gewebeteils eines Auges (10) mittels fokussierter Laserstrahlung, insbesondere zur Durchführung des Verfahrens nach einem der vorhergehenden Ansprüche, umfassend
- eine auf das Auge aufzusetzende Saugringeinheit (16) mit einer Ringachse (22), wobei die Saugringeinheit (16) einen sich in axialer Richtung öffnenden Einführtrichter bildet,
- eine von der Saugringeinheit gesonderte, längs der Ringachse in Koppelkontakt mit dieser bewegbare mechanische Schnittstelleneinheit (34) mit einem Kontaktglas (40) zur Formung der Oberfläche des Auges, wobei die Schnittstelleneinheit (34) einen Konusabschnitt zum axialen Einsetzen in den Einführtrichter aufweist,
- Pumpmittel (88, 90) zur Evakuierung einer zwischen der Saugringeinheit (16), der Schnittstelleneinheit (34) und der Augenoberfläche begrenzten ersten Saugkammer (42),
- eine Sensorik (50, 86) zur Erfassung einer vorgegebenen ersten axialen Relativstellung der Schnittstelleneinheit (34) und der Saugringeinheit (16), wobei in der ersten axialen Relativstellung die Schnittstelleneinheit (34) mit ihrem Konusabschnitt in den Einführtrichter der Saugringeinheit (16) eingetaucht ist,
- eine mit der Sensorik und den Pumpmitteln verbundene Steuereinrichtung (72), welche dazu eingerichtet ist, in Antwort auf die Erfassung der ersten Relativstellung durch die Sensorik eine Evakuierung der ersten Saugkammer (42) zu bewirken, um dadurch einen Kontakt zwischen dem Auge und einer Formungsanlagefläche des Kontaktglases herzustellen oder einen Bereich bestehenden Kontakts zu vergrößern, wobei die Evakuierung der ersten Saugkammer (42) eine relative Bewegung der Schnittstelleneinheit (34) und der Saugringeinheit (16) in eine zweite Relativstellung bewirkt, in der sie stärker einander angenähert sind als in der ersten Relativstellung.

10. Vorrichtung nach Anspruch 9, **dadurch gekennzeichnet, dass** die Saugringeinheit (16) eine ringförmig umlaufende erste Dichtfläche (26) bildet, mit welcher sie zur Abdichtung der ersten Saugkammer (42) an die Augenoberfläche anlegbar ist, wobei die Evakuierung der ersten Saugkammer eine Anlage des Auges an das Kontaktglas (40) in einem Bereich bewirkt, der angenähert der von der ersten Dichtfläche umschlossenen Fläche entspricht.

11. Vorrichtung nach Anspruch 10, **dadurch gekennzeichnet, dass** die erste Dichtfläche (26a) am Innenrand eines an der Saugringeinheit (16a) gebildeten, zum Auge hin abstehenden Ringscheibenkörpers (96a) gebildet ist, welcher die erste Saugkammer (42a) von einer vollständig zwischen der Saugringeinheit (16) und der Augenoberfläche begrenzten, mit der ersten Saugkammer unverbundenen und von dieser unabhängig evakuierbaren zweiten Saugkammer (30a) trennt.

12. Vorrichtung nach einem der Ansprüche 9 bis 11, **dadurch gekennzeichnet, dass** bei ordnungsgemäß auf das Auge aufgesetzter Saugringeinheit (16) in der ersten Relativstellung die erste Saugkammer (42) vor ihrer Evakuierung vollständig an die augenzugewandte Unterseite des Kontaktglases (40) grenzt.

13. Vorrichtung nach einem der Ansprüche 9 bis 12, **dadurch gekennzeichnet, dass** die erste Saugkammer (42) axial in Richtung von dem Auge weg über das Kontaktglas (40) hinausreicht.

14. Vorrichtung nach einem der Ansprüche 9 bis 13, **dadurch gekennzeichnet, dass** der Einführtrichter (20) und der Konusabschnitt zusammenwirkende zweite Dichtflächen zur Abdichtung der ersten Saugkammer (42) bilden.

15. Vorrichtung nach einem der Ansprüche 9 bis 14, **dadurch gekennzeichnet, dass** der Einführtrichter (20) oder/und der Konusabschnitt eine umlaufende Nut (100a) besitzen, in welche ein Dichtring eingesetzt ist.

16. Vorrichtung nach einem der Ansprüche 9 bis 15, **dadurch gekennzeichnet, dass** die Schnittstelleneinheit (34a) einen zur mechanischen Kopplung mit der Saugringeinheit (16a) ausgebildeten, das Kontaktglas (40a) tragenden Kontaktglashalter (102a) sowie einen von dem Kontaktglashalter gesonderten, fest, jedoch auswechselbar mit diesem verbindbaren Adapter (104a) umfasst, welcher mit Koppelformationen (110a) zur Ankopplung an eine Fokussieroptik einer die Laserstrahlung bereitstellenden Lasereinrichtung ausgeführt ist.

17. Vorrichtung nach Anspruch 16, **dadurch gekennzeichnet, dass** der Kontaktglashalter (102a) und der Adapter (104a) mit Gewindemitteln ausgeführt sind, welche eine Schraubverbindung des Kontaktglashalters und des Adapters gestatten.

18. Vorrichtung nach Anspruch 16 oder 17, **dadurch gekennzeichnet, dass** der Kontaktglashalter (102a) in Form einer Konushülse ausgebildet ist.

19. Vorrichtung nach einem der Ansprüche 9 bis 18, **dadurch gekennzeichnet, dass** zumindest die augenzugewandte Seite des Kontaktglases (40) als Planfläche ausgeführt ist.

## Claims

1. A method for coupling a mechanical interface unit (34) of a laser device to a suction ring unit (16) held on an eye tissue by suction force, wherein the suction ring unit comprises a ring axis (22) and forms an insertion funnel which opens in a direction axially away from the eye, the interface unit (34) including a conic section for the purpose of axial insertion into the insertion funnel, comprising the steps:
- relative approaching the interface unit (34) to the suction ring unit (16) as far as into a first relative position, in which the interface unit (34) with its conic section is inserted in the insertion funnel of the suction ring unit (16),
- evacuating a suction chamber (42) formed between the interface unit (34), the suction ring unit (16) and the surface of the tissue in the first relative position, in order thereby to establish a contact between the tissue and a tissue-abutment surface of the interface unit (34) or to enlarge a region of existing contact,
wherein the evacuation of the suction chamber (42) effects a relative movement of the interface unit (34) and the suction ring unit (16) into a second relative position in which they are closer to each other than in the first relative position.

2. The method according to Claim 1, **characterised in that** the evacuation of the suction chamber (42) effects an enlargement of the region of contact between the tissue and the tissue-abutment surface to at least one and a half times, preferably to at least double, in particular to a multiple.

3. The method according to Claim 1 or 2, **characterised in that** the region of contact between the tissue and the tissue-abutment surface is approximately circular-disc-shaped and the evacuation of the suction chamber (42) effects an enlargement of the diameter of the region of contact by at least 30 per cent, preferably by at least 50 per cent, still more preferably by at least 70 per cent, and most preferably by at least 90 per cent.

4. The method according to one of the previous claims, **characterised in that** the relative approach of the interface unit (34) and the suction ring unit (16) is performed in a direction (38) along which a further relative approach of the interface unit (34) and the suction ring unit (16) is possible beyond the first relative position, in particular as far as into a state of mutual abutment.

5. The method according to one of the previous claims, **characterised in that** the path of relative movement of the interface unit (34) and the suction ring unit (16) between the first and the second relative positions amounts to no more than 1 mm.

6. The method according to one of the previous claims, **characterised in that** a proportion of the enlargement of the region of contact between the tissue and the tissue-abutment surface, which is attributable to the relative approach of the interface unit (34) and of the suction ring unit (16) from the first into the second relative position is small, in particular negligibly small, in comparison to a proportion which is attributable to sucking of the tissue against the tissue-abutment surface.

7. The method according to one of the previous claims, **characterised in that** arriving in the first relative position is detected by means of sensorics and **in that** the evacuation of the suction chamber (42) is effected automatically, in particular in a program-controlled manner, in response to the detection of the first relative position.

8. The method according to one of the previous claims, **characterised in that** the relative approach of the interface unit (34) to the suction ring unit (16) is effected by actuation of a motorised drive device (84) and **in that** the operation of the drive device is stopped automatically, in particular in a program-controlled manner, in response to a sensoric detection of the first relative position.

9. An apparatus for cutting a tissue part of an eye (10) by means of focused laser radiation, in particular for the purpose of implementing the method according to one of the preceding claims, comprising
- a suction ring unit (16) to be placed onto the eye and having a ring axis (22), wherein the suction ring unit (16) forms an insertion funnel that opens in an axial direction,
- a mechanical interface unit (34) which is separate from the suction ring unit and movable along the ring axis in coupling contact with the latter and having a contact glass (40) for shaping the surface of the eye, wherein the interface unit (34) comprises a conic section for the axial insertion into the insertion funnel,
- pumping means (88, 90) for evacuating a first suction chamber (42) which is defined between the suction ring unit (16), the interface unit (34) and the surface of the eye,
- sensorics (50, 86) for detecting a predetermined first axial relative position of the interface unit (34) and of the suction ring unit (16), wherein the interface unit (34) with its conic section is inserted in the suction ring unit (16) in the first axial relative position,
- a control device (72) connected to the sensorics and the pumping means and adapted to effect the evacuation of the first suction chamber (42) in response to detection of the first relative position by the sensorics, in order to establish contact between the eye and a shaping abutment surface of the contact glass, or to enlarge a region of existing contact, wherein the evacuation of the first suction chamber (42) causes a relative movement of the interface unit (34) and of the suction ring unit (16) into a second relative position in which they are closer to each other than in the first relative position.

10. The apparatus according to Claim 10, **characterised in that** the suction ring unit (16) forms an annular full-perimeter first sealing surface (26), with which it may be applied to the surface of the eye for the purpose of sealing the first suction chamber (42), whereby the evacuation of the first suction chamber effects an abutment of the eye against the contact glass (40) in a region that corresponds approximately to the area surrounded by the first sealing surface.

11. The apparatus according to Claim 10, **characterised in that** the first sealing surface (26a) is formed on the inner edge of am annular disc body (96a) formed on the suction ring unit (16a) and projecting towards the eye, which separates the first suction chamber (42a) from a second suction chamber (30a) which is completely defined between the suction ring unit (16) and the surface of the eye, is not connected to the first suction chamber, and is capable of being evacuated independently of said first suction chamber.

12. The apparatus according to one of Claims 9 to 11, **characterised in that** with the suction ring unit (16) properly placed onto the eye in the first relative position, the first suction chamber (42) completely borders the underside of the contact glass (40) facing towards the eye, prior to its evacuation.

13. The apparatus according to one of Claims 9 to 12, **characterised in that** the first suction chamber (42) extends axially beyond the contact glass (40) in a direction away from the eye.

14. The apparatus according to one of Claims 9 to 13, **characterised in that** insertion funnel (20) and the conic section form interacting second sealing surfaces for sealing the first suction chamber (42).

15. The apparatus according to one of Claims 9 to 14, **characterised in that** the insertion funnel (20) or/and the conic section form a full-perimeter groove (100a) into which a sealing ring has been inserted.

16. The apparatus according to one of Claims 9 to 15, **characterised in that** the interface unit (34a) includes a contact glass holder (102a) designed for mechanical coupling with the suction-ring unit (16a) and carrying the contact glass (40a) as well as an adapter (104a) which is separate from the contact glass holder and firmly but interchangeably connected to the latter and which is constructed with coupling structures (110a) for coupling to focusing optics of a laser device providing the laser radiation.

17. The apparatus according to Claim 16, **characterised in that** the contact glass holder (102a) and the adapter (104a) are constructed with thread means which permit a screw connection of the contact glass holder and the adapter.

18. The apparatus according to Claim 16 or 17, **characterised in that** the contact glass holder (102a) is designed in the form of a cone sleeve.

19. The apparatus according to one of Claims 9 to 18, **characterised in that** at least the side of the contact glass (40) facing towards the eye is implemented as a plane surface.

## Revendications

1. Procédé pour coupler une unité d'interface mécanique (34) d'un dispositif laser et une unité d'anneau de succion (16) maintenue par succion sur un tissu oculaire, cette unité d'anneau de succion présentant un axe d'anneau (22) et formant un entonnoir d'introduction s'ouvrant le long de la direction axiale dans le sens opposé à l'oeil, l'unité d'interface (34) présentant un tronçon conique en vue de son insertion dans ledit entonnoir d'introduction, ce procédé comprenant les étapes suivantes :
- le rapprochement relatif de l'unité d'interface (34) et de l'unité d'anneau de succion (16) jusqu'à atteinte d'une première position relative dans laquelle le tronçon conique de l'unité d'interface (34) se trouve introduit dans l'entonnoir d'introduction de l'unité d'anneau de succion (16),
- dans cette première position relative, la mise sous vide d'une chambre de succion (42) formée entre l'unité d'interface (34), l'unité d'anneau de succion (16) et la surface du tissu oculaire pour établir ainsi un contact entre le tissu oculaire et une surface de contact tissulaire de l'unité d'interface (34) ou agrandir une zone de contact existante,
la mise sous vide de la chambre de succion (42) engendrant un mouvement relatif de l'unité d'interface (34) et de l'unité d'anneau de succion (16) qui les place dans une deuxième position relative dans laquelle elles sont plus rapprochées l'une de l'autre que dans la première position relative.

2. Procédé selon la revendication 1, **caractérisé en ce que** la mise sous vide de la chambre de succion (42) faire s'agrandir au moins une fois et demie, préférentiellement au moins deux fois et plus particulièrement plusieurs fois la zone de contact entre le tissu oculaire et la surface de contact tissulaire.

3. Procédé selon la revendication 1 ou 2, **caractérisé en ce que** la zone de contact entre le tissu oculaire et la surface de contact tissulaire a approximativement la forme d'un disque circulaire et la mise sous vide de la chambre de succion (42) fait s'agrandir d'au moins 30 pour cent, de manière préférée d'au moins 50 pour cent, de manière encore plus préférée d'au moins 70 pour cent et de manière encore plus particulièrement préférée d'au moins 90 pour cent le diamètre de la zone de contact.

4. Procédé selon l'une des revendications précédentes, **caractérisé en ce que** le rapprochement relatif de l'unité d'interface (34) et de l'unité d'anneau de succion (16) s'effectue dans une direction (38) le long de laquelle il est possible de faire se rapprocher encore plus l'unité d'interface (34) et l'unité d'anneau de succion (16) qui quittent leur première position relative pour venir en butée mutuelle.

5. Procédé selon l'une des revendications précédentes, **caractérisé en ce que** le chemin de déplacement relatif de l'unité d'interface (34) et de l'unité d'anneau de succion (16) entre la première et la deuxième position relative n'excède pas 1 mm.

6. Procédé selon l'une des revendications précédentes, **caractérisé en ce que** la proportion de l'agrandissement de la zone de contact entre le tissu oculaire et la surface de contact tissulaire due au rapprochement relatif de l'unité d'interface (34) et de l'unité d'anneau de succion (16) qui passent de ce fait de leur première à leur deuxième position relative, est faible, plus particulièrement négligemment faible, par rapport à la proportion due à la succion du tissu oculaire contre la surface de contact tissulaire.

7. Procédé selon l'une des revendications précédentes, **caractérisé en ce que** l'atteinte de la première position relative est détectée par capteur, et la mise sous vide de la chambre de succion (42) générée automatiquement, plus particulièrement par commande programmée en réponse à la détection de la première position relative.

8. Procédé selon l'une des revendications précédentes, **caractérisé en ce que** le rapprochement relatif de l'unité d'interface (34) et de l'unité d'anneau de succion (16) est généré par actionnement d'un dispositif d'entraînement (84) motorisé et **en ce que** le fonctionnement dudit dispositif d'entraînement est stoppé automatiquement, plus particulièrement par commande programmée en réponse à la détection de la première position relative.

9. Dispositif pour inciser le tissu d'un oeil (10) à l'aide d'un rayonnement laser focalisé, en particulier pour exécuter le procédé selon l'une des revendications précédentes, lequel dispositif comprend
- une unité d'anneau de succion (16) à poser sur l'oeil et présentant un axe d'anneau (22), cette unité d'anneau de succion (16) formant un entonnoir d'introduction s'ouvrant dans la direction axiale,
- une unité d'interface mécanique (34) déplaçable le long dudit axe d'anneau, réalisée séparément de l'unité d'anneau de succion (16) avec laquelle elle est en contact de couplage, et pourvue d'un verre de contact (40) pour modifier la forme de la surface de l'oeil, cette unité d'interface (34) présentant un tronçon conique pour son insertion axiale dans l'entonnoir d'introduction,
- des moyens de pompage (88, 90) pour réaliser le vide dans la première chambre de succion (42) formée entre l'unité d'anneau de succion (16), l'unité d'interface (34) et la surface de l'oeil,
- un dispositif à capteurs (50, 86) destiné à la détection d'une première position relative axiale prédéterminée de l'unité d'interface (34) et de l'unité d'anneau de succion (16), l'unité d'interface (34) étant plongée avec son tronçon conique dans l'entonnoir d'introduction de l'unité d'anneau de succion (16) lorsqu'elle se trouve dans sa première position relative,
- un dispositif de commande (72) relié au dispositif à capteurs et aux moyens de pompage, lequel dispositif de commande est conçu pour déclencher la mise sous vide de la première chambre de succion (42) en réponse à la détection par le dispositif à capteurs de la première position relative pour établir ainsi un contact entre l'oeil et une surface de contact du verre de contact ou pour agrandir une zone de contact existant, la mise sous vide de la première chambre de succion (42) engendrant un mouvement relatif de l'unité d'interface (34) et de l'unité d'anneau de succion (16) qui les fait se placer dans une deuxième position relative, dans laquelle elles sont plus rapprochées l'un de l'autre que dans la première position relative.

10. Dispositif selon la revendication 9, **caractérisé en ce que** l'unité d'anneau de succion (16) forme une première surface d'étanchéité (26) périphérique de forme annulaire, par le biais de laquelle elle peut être posée sur la surface de l'oeil pour assurer l'étanchéité de la première chambre de succion (42), la mise sous vide de la première chambre de succion entraînant l'application de l'oeil contre le verre de contact (40) dans une zone qui correspond approximativement à la surface couverte par la première surface d'étanchéité.

11. Dispositif selon la revendication 10, **caractérisé en ce que** la première surface d'étanchéité (26a) est réalisée sur le bord intérieur d'un corps formant rondelle (96a) réalisé sur l'unité d'anneau de succion (16a) et faisant saillie en direction de l'oeil, lequel corps sépare la première chambre de succion (42a) d'une deuxième chambre de succion (30a) qui est parfaitement délimitée entre l'unité d'anneau de succion (16) et la surface de l'oeil, n'est pas reliée à la première chambre de succion et peut être mise sous vide indépendamment de cette dernière.

12. Dispositif selon l'une des revendications 9 à 11, **caractérisé en ce qu'**avant sa mise sous vide, la première chambre de succion (42) confine entièrement à la face inférieure du verre de contact (40) tournée vers l'oeil lorsque l'unité d'anneau de succion (16) est correctement posée sur l'oeil, dans la première position relative.

13. Dispositif selon l'une des revendications 9 à 12, **caractérisé en ce que** la première chambre de succion (42) va axialement au-delà du verre de contact (40) dans une direction qui l'éloigne de l'oeil.

14. Dispositif selon l'une des revendications 9 à 13, **caractérisé en ce que** l'entonnoir d'introduction (20) et le tronçon conique forment des deuxièmes surfaces d'étanchéité interagissant entre elles pour assurer l'étanchéité de la première chambre de succion (42).

15. Dispositif selon l'une des revendications 9 à 14, **caractérisé en ce que** l'entonnoir d'introduction (20) ou/et le tronçon conique possède(nt) une gorge (100a) périphérique dans laquelle est placé un joint annulaire.

16. Dispositif selon l'une des revendications 9 à 15, **caractérisé en ce que** l'unité d'interface (34a) comporte un porte-verre de contact (102a) supportant le verre de contact (40a) et conçu pour assurer le couplage mécanique avec l'unité d'anneau de succion (16), ainsi qu'un adaptateur (104a) réalisé séparément du porte-verre de contact et pouvant être relié à ce dernier de façon fixe mais amovible, lequel adaptateur est réalisé pourvu de formations de couplage (110a) pour le couplage avec une optique de focalisation d'un dispositif laser destiné à fournir le rayonnement laser.

17. Dispositif selon la revendication 16, **caractérisé en ce que** le porte-verre de contact (102a) et l'adaptateur (104a) sont réalisés pourvus de filetages lesquels permettent un assemblage vissé du porte-verre de contact et de l'adaptateur.

18. Dispositif selon la revendication 16 ou 17, **caractérisé en ce que** le porte-verre de contact (102a) est réalisé sous la forme d'une douille conique.

19. Dispositif selon l'une des revendications 9 à 18, **caractérisé en ce qu'**au moins le côté du verre de contact (40) tourné vers l'oeil est réalisé sous la forme d'une surface plane.
